Europäisches Patentamt

European Patent Office

Office européen des brevets

(19)

(11) EP 0 725 050 B1

(12) EUROPÄISCHE PATENTSCHRIFT

(45) Veröffentlichungstag und Bekanntmachung des Hinweises auf die Patenterteilung:
**30.12.1998 Patentblatt 1998/53**

(51) Int. Cl.$^6$: **C07C 17/20**, C07C 25/13

(21) Anmeldenummer: **96100683.0**

(22) Anmeldetag: **18.01.1996**

(54) **Verfahren zur Herstellung von kernhalogenierten Benzotrichloriden aus den entsprechenden Benzotrifluoriden**

Process for the preparation of nuclear halides of benzotrichlorides from the corresponding benzotrifluorides

Procédé pour la préparation d'halogénures nucléaires de trichlorures de benzyle à partir des trifluorures de benzyle correspondants

(84) Benannte Vertragsstaaten:
**BE CH DE ES FR GB IE IT LI NL**

(30) Priorität: **31.01.1995 DE 19502942**

(43) Veröffentlichungstag der Anmeldung:
**07.08.1996 Patentblatt 1996/32**

(73) Patentinhaber: BAYER AG
**51368 Leverkusen (DE)**

(72) Erfinder:
- **Döring, Fritz**
  **D-51519 Odenthal (DE)**
- **Gehring, Reinhold, Dr.**
  **D-42327 Wuppertal (DE)**
- **Heinrich, Josef, Dr.**
  **D-42719 Solingen (DE)**

(56) Entgegenhaltungen:
DE-A- 2 546 533          DE-A- 4 301 247
US-A- 1 973 069

**Beschreibung**

Die vorliegende Erfindung betrifft ein besonders vorteilhaftes Verfahren zur Herstellung von kernhalogenierten Benzotrichloriden aus den entsprechenden Benzotrifluoriden.

Aus der DE-A 43 01 247 ist bekannt, daß man bestimmte kernhalogenierte Benzotrifluoride mit Chloriden aus der Reihe der Friedel-Crafts-Katalysatoren umsetzen und so die entsprechenden kernhalogenierten Benzotrichloride erhalten kann. Als geeignete Chloride werden Aluminiumtrichlorid, Titantetrachlorid, Antimonpentachlorid und Bortrichlorid angegeben. Diese werden in Mengen von 1 bis 1,5 Mol pro Mol Benzotrifluorid eingesetzt. Nachteilig bei diesem Verfahren ist der notwendige Umgang mit chlorierten Lösungsmitteln und die damit verbundene geringe Raum-Zeit-Ausbeute. Außerdem müssen die Chloride aus der Reihe der Friedel-Crafts-Katalysatoren im Überschuß eingesetzt werden, was zu schwierig entsorgbaren Rückständen führt.

Es wurde nun ein Verfahren zur Herstellung von kernhalogenierten Benzotrichloriden aus den entsprechenden kernhalogenierten Benzotrifluoriden gefunden, das dadurch gekennzeichnet ist, daß man kernhalogenierte Benzotrifluoride mit Siliciumtetrachlorid in Gegenwart katalytischer Mengen Aluminiumtrichlorid umsetzt.

Zum Einsatz in das erfindungsgemäße Verfahren sind beispielsweise chlorierte und/oder fluorierte Benzotrifluoride geeignet. Vorzugsweise werden 2,3,4,5-Tetrafluor-, 2,3,5,6-Tetrafluor-, 3-Chlor-2,4,5-trifluor- oder 4-Chlor-benzotrifluorid eingesetzt, besonders bevorzugt 2,3,4,5-Tetrafluorbenzotrifluorid. Die einzusetzenden kernhalogenierten Benzotrifluoride sind bekannte Verbindungen oder analog zu bekannten Verbindungen zugänglich.

Siliciumtetrachlorid kann in handelsüblicher Qualität eingesetzt werden. Pro Mol eingesetztes kernhalogeniertes Benzotrifluorid kann man z.B. 0,7 bis 1,5 Mol Siliciumtetrachlorid einsetzen. Vorzugsweise liegt diese Menge bei 0,75 bis 1,0 Mol, besonders bevorzugt bei 0,75 Mol. Größere Überschüsse an Siliciumtetrachlorid sind im allgemeinen nicht grundsätzlich von Nachteil, jedoch aus wirtschaftlichen Überlegungen uninteressant.

Aluminiumtrichlorid kann man pro Mol eingesetztes kernhalogeniertes Benzotrifluorid z.B. in Mengen von 0,01 bis 0,2 Mol einsetzen. Vorzugsweise liegt diese Menge bei 0,05 bis 0,15 Mol.

Man kann das erfindungsgemäße Verfahren z.B. bei Temperaturen im Bereich 0 bis 80°C durchführen. Bevorzugt sind Temperaturen im Bereich 15 bis 60°C. Beim Einsatz von in p-Position Halogenatome enthaltenden kernhalogenierten Benzotrifluoriden sind Temperaturen im Bereich 15 bis 40°C bevorzugt.

Der während der Durchführung des erfindungsgemäßen Verfahrens herrschende Druck ist nicht kritisch. Man kann bei Normaldruck, Unterdruck oder Überdruck arbeiten. Vorzugsweise arbeitet man bei Normaldruck.

Während des Ablaufs der erfindungsgemäßen Umsetzung tritt aus dem Reaktionsgemisch Siliciumtetrafluorid aus. Mit diesem kann man auf unterschiedliche Weise verfahren. Beispielsweise kann man es in wäßrige Natronlauge einleiten, wobei es dann allerdings für eine Weiterverwendung im allgemeinen nicht mehr brauchbar ist. Man kann es auch in Fluorwasserstoff absorbieren, wobei man Hexafluorokieselsäure erhält, die man z.B. in an sich bekannter Weise, z.B. als Konservierungs- oder Reinigungsmittel, verwenden kann. Weiterhin kann man Siliciumtetrafluorid auch in einem Alkohol auffangen, daraus das Siliciumtetrafluorid wiedergewinnen und in an sich bekannter Weise, z.B. zur Herstellung von hochdispersem Siliciumdioxid oder zur Erhöhung der Beständigkeit von Beton (Ocart-Verfahren) verwenden.

Die erfindungsgemäße Umsetzung kann in aus üblichen Werkstoffen hergestellten Apparaturen durchgeführt werden. Geeignete Werkstoffe für eine Durchführung im technischen Maßstab sind z.B. Edelstähle.

Nach Durchführung des erfindungsgemäßen Verfahrens liegt im allgemeinen ein Reaktionsgemisch vor, das neben dem gewünschten Reaktionsprodukt eine Aluminiumsalze enthaltende Masse und gegebenenfalls überschüssiges Siliciumtetrachlorid enthält. Überschüssiges Siliciumtetrachlorid kann man z.B. durch Destillation abtrennen und in einem weiteren Ansatz wieder einsetzen. Die Aluminiumsalze enthaltende Masse kann man vom hergestellten kernhalogenierten Benzotrichlorid z.B. durch Filtration abtrennen. Die abgetrennte Aluminiumsalze enthaltende Masse kann anstelle von frischem Aluminiumtrichlorid in einem weiteren Ansatz wieder eingesetzt werden. Bei wiederholter Rückführung der Aluminiumsalze enthaltenden Masse ist es vorteilhaft von Zeit zu Zeit frisches Aluminiumtrichlorid hinzuzufügen, z.B. nach jeder 2. bis 5. Rückführung z.B. 10 bis 25 Gew.-% der ursprünglich eingesetzten Aluminiumtrichlorid-Menge.

Das Filtrat aus der Abtrennung der Aluminiumsalze enthaltenden Masse besteht häufig aus hochreinem halogeniertem Benzotrichlorid, das direkt weiter verwendet werden kann. Gewünschtenfalls kann man das kernhalogenierte Benzotrichlorid weiter reinigen, z.B. durch Destillation, vorzugsweise unter vermindertem Druck.

Kernhalogenierte Benzotrichloride sind wichtige Zwischenprodukte zur Herstellung von Chinoloncarbonsäurederivaten, die antibakterielle Wirkstoffe sind (siehe z.B. DE-A 3 420 796 und EP-A 417 669).

Neben hohen Ausbeuten im Bereich von 90 % und mehr hat das erfindungsgemäße Verfahren eine Reihe weiterer Vorteile. So wird nur mit katalytischen Mengen Aluminiumtrichlorid gearbeitet. Durch die Rückführungsmöglichkeit entstehen nur sehr geringe Mengen zu entsorgender Abfälle. Die Raum-Zeit-Ausbeuten sind hoch, da lösungsmittelfrei gearbeitet wird. Die Aufarbeitung der Reaktionsgemische ist sehr einfach, da das Nebenprodukt Siliciumtetrafluorid gasförmig aus dem Reaktionsgemisch entweicht.

## Beispiele

### Beispiel 1

Herstellung von 4-Chlorbenzotrichlorid

In einem 1 l-Rührkolben, versehen mit Tropftrichter und Rückflußkühler wurden 13,5 g Aluminiumtrichlorid (wasserfrei) und 180,5 g 4-Chlorbenzotrifluorid vorgelegt und die Mischung auf 50°C erwärmt. Unter Rühren wurden im Verlauf von 2 Stunden 170 g Siliziumtetrachlorid zugetropft. Das bei der Reaktion anfallende Siliziumtetrafluorid-Abgas wurde unter Zumischung von Stickstoff in einen Polyethylen-Rührtopf auf verdünnte, wäßrige Natronlauge geleitet. Entsprechend der Menge des eingeleiteten Abgases wurde laufend verdünnte wäßrige Natronlauge (ca. 2,5 %-ig) mittels Pumpe nachdosiert und über einen Siphon am Boden des Gefäßes kontinuierlich ein Ablauf entnommen. Nach Beendigung der Zugabe des Siliziumtetrachlorids wurde 3 Stunden nachgerührt und anschließend das Reaktionsgemisch im Vakuum destilliert bis trockenes Aluminiumsalz vorlag.

Ergebnis:

1. Fraktion, Druck 1.013 bis 1.015 mbar, 31 g Siliziumtetrachlorid
2. Fraktion, Siedepunkt 112 bis 115°C/15 mbar, 208 g 4-Fluorbenzotrichlorid
(Gehalt nach GC 99%, $n_D^{20}$ = 1,5712, Ausbeute: 89,5 % d. Theorie)

### Beispiel 2

Herstellung von 2,3,4,5-Tetrafluorbenzotrichlorid

Es wurde gearbeitet wie in Beispiel 1 beschrieben. Vorgelegt wurden 20 g Aluminiumtrichlorid (wasserfrei) und 218 g 2,3,4,5-Tetrafluorbenzotrifluorid (96 %-ig). Anschließend wurden bei 50°C unter Rühren innerhalb von 2 Stunden 170 g Siliziumtetrachlorid zugetropft. Die Destillation ergab:

1. Fraktion, Druck 1.013 bis 20 mbar, 35 g Siliziumtetrachlorid
2. Fraktion, Siedepunkt 50 bis 55°C/4 mbar, 238 g 2,3,4,5-Tetrafluorbenzotrichlorid
(Ausbeute: 88,9 % d. Theorie)

### Beispiel 3

Herstellung von 2,4,5-Trifluor-3-chlorbenzotrichlorid

Es wurde gearbeitet wie in Beispiel 1 beschrieben. Vorgelegt wurden 234,5 g 2,4,5-Trifluor-3-chlorbenzotrifluorid (98 %-ig) und 13,5 g Aluminiumtrichlorid (wasserfrei). Anschließend wurden bei 50 bis 55°C innerhalb von 1,5 Stunden 70 g Siliziumtetrachlorid zugetropft und noch 4 Stunden bei dieser Temperatur nachgerührt. Die Destillation ergab:

1. Fraktion, Druck 1.013 bis 20 mbar, 30 g Siliziumtetrachlorid
2. Fraktion, Siedepunkt 72 bis 76°C/2 mbar, 274 g 2,4,5-Trifluor-3-chlorbenzotrichlorid
(Gehalt nach GC 97 %, Ausbeute: 95,5 % d. Theorie)

## Patentansprüche

1. Verfahren zur Herstellung von kernhalogenierten Benzotrichloriden aus den entsprechenden kernhalogenierten Benzotrifluoriden, dadurch gekennzeichnet, daß man kernhalogenierte Benzotrifluoride mit Siliciumtetrachlorid in Gegenwart katalytischer Mengen Aluminiumtrichlorid umsetzt.

2. Verfahren nach Anspruch 1, dadurch gekennzeichnet, daß man 2,3,4,5-Tetrafluor-, 2,3,5,6-Tetrafluor-, 3-Chlor-2,4,5-trifluor- oder 4-Chlor-benzotrifluorid einsetzt.

3. Verfahren nach Ansprüchen 1 und 2, dadurch gekennzeichnet, daß man pro Mol eingesetztes Benzotrifluorid 0,7 bis 1,5 Mol Siliziumtetrachlorid einsetzt.

4. Verfahren nach Ansprüchen 1 bis 3, dadurch gekennzeichnet, daß man pro Mol eingesetztes halogeniertes Benzotrifluorid 0,01 bis 0,2 Mol Aluminiumtrichlorid einsetzt.

5. Verfahren nach Ansprüchen 1 bis 4, dadurch gekennzeichnet, daß man es bei Temperaturen im Bereich 0 bis 80°C durchführt.

6. Verfahren nach Ansprüchen 1 bis 5, dadurch gekennzeichnet, daß man beim Einsatz von in p-Position Halogenatome enthaltenden halogenierten Benzotrifluoriden bei Temperaturen im Bereich 15 bis 40°C arbeitet.

7. Verfahren nach Ansprüchen 1 bis 6, dadurch gekennzeichnet, daß man das während der Umsetzung aus dem Reaktionsgemisch austretende Siliciumtetrafluorid in wäßrige Natronlauge einleitet, in Fluorwasserstoff absorbiert oder in einem Alkohol auffangt.

8. Verfahren nach Ansprüchen 1 bis 7, dadurch gekennzeichnet, daß man zur Aufarbeitung des Reaktionsgemisches zunächst gegebenenfalls vorhandenes überschüssiges Siliciumtetrachlorid durch Destillation abtrennt und eine Aluminiumsalze enthaltende Masse vom hergestellten kernhalogenierten Benzotrichlorid durch Filtration abtrennt.

9. Verfahren nach Ansprüchen 1 bis 8, dadurch gekennzeichnet, daß man in die erfindungsgemäße Umsetzung anstelle von frischem Aluminiumtrichlorid die aus dem Reaktionsgemisch eines vorhergehenden Ansatzes abgetrennte Aluminiumsalze enthaltende Masse einsetzt.

## Claims

1. Process for the preparation of nuclear-halogenated benzotrichlorides from the corresponding nuclear-halogenated benzotrifluorides, characterized in that nuclear-halogenated benzotrifluorides are reacted with silicon tetrachloride in the presence of catalytic amounts of aluminium trichloride.

2. Process according to Claim 1, characterized in that 2,3,4,5-tetrafluoro-, 2.3,5,6-tetrafluoro-, 3-chloro-2,4,5-trifluoro- or 4-chloro-benzotrifluoride is employed.

3. Process according to Claims 1 and 2 characterized in that 0.7 to 1.5 mol of silicon tetrachloride are employed per mole of benzotrifluoride employed

4. Process according to Claims 1 to 3, characterized in that 0.01 to 0.2 mol of aluminium trichloride is employed per mole of halogenated benzotrifluoride employed.

5. Process according to Claims 1 to 4, characterized in that it is carried out at temperatures in the range from 0 to 80°C.

6. Process according to Claims 1 to 5, characterized in that if halogenated benzotrifluorides containing halogen atoms in the p-position are employed, it is carried out at temperatures in the range from 15 to 40°C.

7. Process according to Claims 1 to 6, characterized in that the silicon tetrafluoride discharged from the reaction mixture during the reaction is tetrafluoride discharged from the reaction mixture during the reaction is passed into aqueous sodium hydroxide solution, absorbed in hydrogen fluoride or collected in an alcohol.

8. Process according to Claims 1 to 7, characterized in that for working up the reaction mixture, any excess silicon tetrachloride present is first separated off by distillation and a mass comprising aluminium salts is separated off by filtration from the nuclear-halogenated benzotrichloride prepared.

9. Process according to Claims 1 to 8, characterized in that in the reaction according to the invention, the mass comprising aluminium salts which has been separated off from the reaction mixture of a preceding batch is employed instead of fresh aluminium trichloride.

## Revendications

1. Procédé pour la préparation de benzotrichlorures halogénés au noyau à partir des benzotrifluorures correspondants halogénés au noyau, caractérisé en ce qu'on fait réagir des benzotrifluorures halogénés au noyau avec du tétrachlorure de silicium en présence de quantités catalytiques de trichlorure d'aluminium.

2. Procédé selon la revendication 1, caractérisé en ce qu'on met en oeuvre le 2,3,4,5-tétrafluoro-, le 2,3,5,6-tétrafluoro-, le 3-chloro-2,4,5-trifluoro-ou le 4-chloro-benzotrifluorure.

3. Procédé selon les revendications 1 et 2, caractérisé en ce qu'on met en oeuvre, par mole du benzotrifluorure mis en oeuvre, de 0,7 à 1,5 mole de tétrachlorure de silicium.

4. Procédé selon les revendications 1 à 3, caractérisé en ce qu'on met en oeuvre, par mole du benzotrifluorure halogéné mis en oeuvre, de 0,01 à 0,2 mole de trichlorure d'aluminium.

5. Procédé selon les revendications 1 à 4, caractérisé en ce qu'on l'effectue à des températures dans le domaine de 0 à 80°C.

6. Procédé selon les revendications 1 à 5, caractérisé en ce qu'on travaille à des températures dans le domaine de 15 à, 40°C lorsqu'on met en oeuvre des benzotrifluorures halogénés contenant des atomes d'halogène en position p.

7. Procédé selon les revendications 1 à 6, caractérisé en ce que le tétrafluorure de silicium qui s'évacue du mélange réactionnel au cours de la mise en réaction est introduit dans de la soude caustique aqueuse, est absorbé dans de l'acide fluorhydrique ou bien est recueilli dans un alcool.

8. Procédé selon les revendications 1 à 7, caractérisé en ce que, pour le traitement du mélange réactionnel, on sépare d'abord par distillation le tétrachlorure de silicium en excès éventuellement présent et on sépare par filtration du benzotrichlorure obtenu halogéné au noyau une matière contenant des sels d'aluminium.

9. Procédé selon les revendications 1 à 8, caractérisé en ce qu'on met en oeuvre, dans la mise en réaction selon l'invention, au lieu de trichlorure d'aluminium frais, la matière contenant des sels d'aluminium, séparée du mélange réactionnel d'une charge antérieure.